(19) Europäisches Patentamt
European Patent Office
Office européen des brevets

(11) **EP 4 467 975 A1**

(12) **EUROPEAN PATENT APPLICATION**

(43) Date of publication:
**27.11.2024 Bulletin 2024/48**

(21) Application number: **23174755.1**

(22) Date of filing: **23.05.2023**

(51) International Patent Classification (IPC):
**G01N 31/22** (2006.01)    **G01N 21/64** (2006.01)
**G01N 21/78** (2006.01)    **G01N 33/38** (2006.01)

(52) Cooperative Patent Classification (CPC):
**G01N 31/22; G01N 33/383;** C04B 40/0096;
G01N 21/6408

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Katholieke Universiteit Leuven
3000 Leuven (BE)**

(72) Inventor: **The designation of the inventor has not
yet been filed**

Remarks:
Claims 16-19 are deemed to be abandoned due to
non-payment of the claims fees (Rule 45(3) EPC).

(54) **BUILT STRUCTURE MONITOR**

(57) The present invention relates generally to sensing the presence of a list of anions, appearing at the same time or individually, in alkaline materials. More particularly, it concerns sensing and quantifying the presence of carbonate ($CO_3^{2-}$), chloride ($Cl^-$), sulphate ($SO_4^{2-}$), nitrate ($NO_3^-$) and/or nitrite ($NO_2^-$), hereinafter called "anions of interest", in any of 1) calcium carbonate or dolomite-based materials, 2) materials comprising calcium carbonate or dolomite, 3) cement based materials or 4) concrete (for instance reinforced concrete). For instance, such materials can form structural elements of constructions such as bridges or roads so that the present invention also concerns sensing the anions of interest in such constructions.

The present invention relates to (i) the use of a photoluminescent active sensor material to sense the anions of interest in alkaline materials, the method of doing it and (iii) a construction of a device to do it. As an example of realization, the carbonation is hereby measured by the photoluminescence of Eu doped ZnAl layered double hydroxide that took up carbonate from a solution resulting from the carbonation of these materials to be sensed. For instance, the carbonation of reinforced concrete can be measured by the photoluminescence of Eu dope ZnAl layered double hydroxide that took up carbonate from a solution resulting from electrochemical reactions between carbon dioxide, moisture and calcium hydroxide that is present in the concrete.

More particularly the present invention concerns luminescent $Eu^{3+}$ substituted in layered double hydroxide (LDH) layers for use in such remote optical sensor. This was found to enable optical, calibrationless quantification of anionic species in the interlayers for use in an optical sensor to detect intrusion of anions such as $Cl^-$ or $CO_3^{2-}$.

EP 4 467 975 A1

**Description**

**Background and Summary**

**BACKGROUND OF THE INVENTION**

**A. Field of the Invention**

The present invention relates generally to sensing the presence of a list of anions, appearing at the same time or individually, in alkaline materials. More particularly, it concerns sensing and quantifying the presence of carbonate ($CO_3^{2-}$), chloride ($Cl^-$), sulphate ($SO_4^{2-}$), nitrate ($NO_3^-$) and/or nitrite ($NO_2^-$), hereinafter called "anions of interest", in any one material selected of the group consisting of 1) calcium carbonate or dolomite-based materials, 2) materials comprising calcium carbonate or dolomite, 3) cement or cement based materials and 4) concrete (for instance reinforced concrete). For instance, such materials can form structural elements of constructions such as bridges or roads so that the present invention also concerns sensing the anions of interest in such constructions.

**B. Description of the Related Art**

Concrete is the material most produced by mankind (about 30 Gt/y as of 2020), outstripping production volumes of other materials by an order of magnitude. Concrete, mainly Portland cement based, currently represents $\pm60$ % of all man-made materials. Concrete is present in buildings, bridges, nuclear containments, housing, roads, and dams (Herbert Pöllmann, Ruben Snellings, Luca Valentini; Cement and Concrete-Past, Present, and Future. Elements 2022; 18 (5): 295-299).

Corrosion of the reinforcing bar (rebar) in concrete-based structures is one of the dominant mechanisms limiting the lifetime of reinforced concrete constructions (P. C. Association, Types and causes of concrete deterioration, Portl. Cem. Assoc. - Concr. Inf., 2002).

Fresh concrete contains a mixture of alkaline materials, ensuring a pH concrete pore water environment of pH 12 - 13. This pH induces the formation of a passivating layer on the surface of the steel rebar, thus inhibiting corrosion. Carbonation of concrete by the uptake of carbon dioxide ($CO_2$) from the air can cause the passivating layer in steel rebars to shrink. In this mechanism, $CO_2$ reacts with the hydroxides present in the concrete, converting them into metal carbonates and decreasing the local pH. When the low pH front reaches the steel rebar and the steel is depassivated, corrosion kicks thus jeopardising the lifetime of the construction. Aside its own impact in the rebar passivation layer, carbonation also increases the sensitivity of concrete to other corrosion mechanisms such as chloride attack, and thus is a key determinant of the service life of concrete structures. An overview of concrete deterioration pathways, including carbonation, can be read in P. C. Association, Types and causes of concrete deterioration, Portl. Cem. Assoc. - Concr. Inf., 2002.

In the current art of assessing carbonation in concrete, the dominant approach is drilling pieces of concrete from constructions and spraying a phenolphthalein solution on the collected material, thus detecting the local pH to be higher or lower than pH 10 (De Maeijer, P. K., Craeye, B., Snellings, R., Kazemi-Kamyab, H., Loots, M., Janssens, K., & Nuyts, G. (2020). Effect of ultra-fine fly ash on concrete performance and durability. Construction and Building Materials, 263, 120493). This method is described and standardized by the European Standard EN 14630.

However, drilling is detrimental to the concrete structure, demands scaffolding for accessing difficult-to-reach parts of constructions, and demands transport of materials and specialty laboratory equipment, which are current hurdles of the art.

There is thus a need for remote sensing methods and devices that can warn for such carbonation damage without the need for drilling, scaffolding and transport of materials.

The present invention provides such solution by describing, at first, a method that in one instance of its application is used or can be used for the remote sensing of carbonation in concrete (among other materials, more generally, alkaline materials) and, at second, a sensor device that applies this method and realizes the non-destructive remote detection of the progress of the carbonation front in concrete structures. A non-exhaustive realization example for the here described device can be built from lanthanide-doped layered double hydroxides combined with fibre-based detection of their luminescent properties. Embedding such a device in the concrete structure to be monitored solves the problems mentioned hereinabove. The present invention provides a reliable, in situ, remote non-destructive method to characterize the evolution of the carbonation front in concrete structures, among others. The present invention provides a method and a device to acquire data that will be used for the planning of maintenance actions in concrete structures.

**EP 4 467 975 A1**

## SUMMARY OF THE INVENTION

The present invention solves the problems of the related art of monitoring the deterioration of built structures (for instance concrete-based structures) by, with one of its realization examples, remotely and non-destructively detecting and quantifying carbonate in the built structure.

In accordance with the purpose of the invention, as embodied and broadly described herein, the invention is broadly drawn to remotely quantify the local carbonate concentration inside a built structure or part thereof, whereby the built structure or part thereof to be monitored comprises an alkaline material of the group consisting of calcium carbonate or dolomite based materials, materials comprising calcium carbonate or dolomite, cement based materials and concrete, whereby the method comprising said contacting the alkaline material with a fluorescent material containing lanthanide elements and by which the detected luminescence of the fluorescent material can be used to sense the presence of carbonate in the alkaline material.

In one aspect of the invention, the sensor of present invention comprises an $Eu^{3+}$ doped layered double hydroxide (LDH) as a fluorescent material with said features. In a further embodiment of the invention, the sensor is functionally coupled to a fluorimeter to receive the fluorescent signal and the fluorimeter is functionally coupled to a processor when operational to transmit the signal to a processor to process the luminescence decay lifetime and/or the $I(^5D_0 \rightarrow {}^7F_2)/I(^5D_0 \rightarrow {}^7F_1)$ luminescence intensity ratio in the $Eu^{3+}$ doped LDH sensor into a quantification of carbonate load.

Another aspect of the invention concerns that the sensor comprises a fluorimeter coupled to receive the fluorescent signal and a controller coupled to fluorimeter that transmits the signal to a processor to process the luminescence decay lifetime and/or the $I(^5D_0 \rightarrow {}^7F_2)/I(^5D_0 \rightarrow {}^7F_1)$ luminescence intensity ratio in the $Eu^{3+}$ doped LDH sensor into a quantification of carbonate load whereby the carbonate or anion intrusion into a build structure is quantified without reference to an external calibration.

Some embodiments of the invention are set forth in claim format directly below:

1. A method to monitor, to analyse or to quantify the presence of an anion of interest into a built structure or part thereof, whereby the built structure or part thereof to be dosed comprises a cured or hardened material selected of the group consisting of calcium carbonate and dolomite based material, materials comprising calcium carbonate or dolomite, cement based materials and concrete, whereby the method comprising said contacting the built structure or part thereof with an active sensor material comprising a lanthanide doped material of the group consisting of layered double hydroxides (LDH), metal oxides, mixed metal oxides, mixed metal oxyhydroxides and sensing the fluorescent signal thereof to sense the anion of interest.

2. The method according to embodiment 1, whereby the active sensor material comprises $Eu^{3+}$-doped layered double hydroxide (LDH).

3. The method according to any one of the embodiments 1 and 2, whereby the fluorescent signal sensor is an optical sensor.

4. The method according to any one of the embodiments 1 to 3, whereby the sensor further comprising a fluorimeter coupled to receive the fluorescent signal and a controller coupled to fluorimeter that transmits the signal to a processor to process the luminescence decay lifetime and/or the luminescence intensity in the sensor into a quantification of load of the anion of interest.

5. The method according to any one of the embodiments 1 to 3, whereby the sensor further comprising a fluorimeter coupled to receive the fluorescent signal and a controller coupled to fluorimeter that transmits the signal to a processor to process the luminescence decay lifetime and/or the $I(^5D_0 \rightarrow {}^7F_2)/I(^5D_0 \rightarrow {}^7F_1)$ luminescence intensity ratio in the active sensor into a quantification of carbonate load whereby the anion intrusion into a built structure is quantified without reference to an external calibration

6. The method according to any one of the embodiments 1 to 5, whereby the cured or hardened material is cured or hardened reinforced concrete.

7. The method according to any one of the embodiments 1 to 5, whereby the cured or hardened material is cured or hardened reinforced concrete comprising Portland cement.

8. The method according to any one of the embodiments 1 to 5, whereby the anion of interest is any one of the group consisting of carbonate ($CO_3^{2-}$), chloride ($Cl^-$), sulphate ($SO_4^{2-}$), nitrate ($NO_3^-$) and nitrite ($NO_2^-$).

9. The method according to any one of the embodiments 1 to 5, whereby the anion of interest is any one of the group consisting of carbonate $F^-$, $Br^-$, $I^-$, $I^{2-}$, $I^{3-}$, oxyanions of B, C, N, P, S, Cr, Mn, Ge, As, Se, Nb, Tc, Br, I, W, Re, Os, Ir, Pt, Hg and oxyanions of actinide elements (Th-Lr).

10. The method according to any one of the embodiments 1 to 5, whereby the intruding anions are of the group of $Cl^-$ and $CO_3^{2-}$ or a combination thereof.

11. The method according to any one of the embodiments 1 to 5, whereby the anion of interest is carbonate ($CO_3^{2-}$) and whereby a processor quantifies the carbonation degree by the amount of carbonate taken up by an $Eu^{3+}$-doped active sensor material by the equation: *wt% C* = 0.75 × ($I_{21}$ - 2.8), where $I_{21}$ = $I(^5D_0 \rightarrow {}^7F_2)/I(^5D_0 \rightarrow {}^7F_1)$ is the ratio

between the integral intensities of the $^5D_0\rightarrow^7F_2$ and $^5D_0\rightarrow^7F_1$ emission bands of $Eu^{3+}$ in an Eu-containing active sensor material. By carbonate uptake, wt%C increases ranging from 0 to approximately 2%, this maximum figure meaning that the sensor is saturated.

12. The method according to any one of the embodiments 1 to 11, to monitor the changing chemical environment of the built structure during monitoring session of years to analyse the integrity of the built structure.

13. The method according to any one of the embodiments 1 to 11, to monitor the changing chemical environment of the built structure during monitoring session of decades to analyse the integrity of the built structure.

14. The method according to any one of the embodiments 1 to 11, to monitor the changing chemical environment of the built structure.

15. The method according to any one of the embodiments 1 to 11, to monitor the built structure by measuring absolute luminescence intensities.

16. The method according to any one of the embodiments 1 to 11, to monitor the built structure by measuring absolute luminescence intensities and calculating luminescence lifetimes from consequent measurements.

17. The method according to any one of the embodiments 1 to 11, whereby the active sensor contact said cured or hardened built structure to take up carbonate that is present in the cured or hardened material and the resulting photoluminescence of the active sensor material is measured.

18. The method according to any one of the embodiments 1 to 17, whereby the active sensor is embedded in the cured or hardened material of the built structure.

19. The method according to any one of the embodiments 1 to 18, whereby the active sensor is functionally connected with optical fibres which are connected or connectable with a fluorimeter.

20.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

## Detailed Description

### DETAILED DESCRIPTION OF EMBODIMENTS OF THE INVENTION

The following detailed description of the invention refers to the accompanying drawings. The same reference numbers in different drawings identify the same or similar elements. Also, the following detailed description does not limit the invention. Instead, the scope of the invention is defined by the appended claims and equivalents thereof.

Several documents are cited throughout the text of this specification. Each of the documents herein (including any manufacturer's specifications, instructions etc.) are hereby incorporated for reference; however, there is no admission that any document cited is indeed prior art of the present invention.

**The following definitions are used herein:**
As used herein, the term "Cement-based materials" refers to composite materials composed primarily of cement, aggregates (such as sand or gravel), and water. Cement is made by heating a mixture of limestone and clay or other materials at high temperatures. The most common type of cement used in construction is Portland cement. Cement-based materials are widely used in the construction industry for making concrete, mortar, and other structural elements. Hereby cement comprises hydraulic cements that will be cured or will be hardened to make the built structure and such cement comprises any one of the group consisting of Portland Cements, Blended Cements, Calcium Sulfoaluminate-based Cements, Calcium Aluminate Cements, Belite Cements, Alkali-activated Slag based Cements, Alkali-activated Portland Blended Cements, Alkali-activated pozzolan cements, Alkali-activated lime-pozzolan/slag cements, Alkali-activated calcium aluminate blended cements, Magnesia Silicate Cements and Sorel Cements or a combination thereof.

As used herein, the term "Concrete" refers to a composite material composed of cement, aggregates, and water. It is one of the most widely used construction materials globally.

The aggregates used in concrete can include sand, gravel, crushed stone, or recycled materials. When mixed with water, cement undergoes a chemical reaction known as hydration, which hardens the mixture and binds the aggregates together. Concrete is valued for its strength, durability, and versatility, making it suitable for various construction applications such as buildings, roads, bridges, and dams.

As used herein "active sensor" refers to a sensing material, devices or systems that emit energy or signals to detect and measure objects or phenomena. Unlike passive sensors that rely on external sources of energy, active sensors generate their own energy to illuminate the target and then measure the response.

As used herein, the term "hydraulic cement" refers to cement that sets and hardens in the presence of water. In various embodiments, the hydraulic cement binder is selected from the group consisting of Portland cement, blended Portland cement, expansive cement, rapid setting and hardening cement, calcium aluminate cement, magnesium phosphate, and mixtures thereof. In specific embodiments, the hydraulic cement binder comprises or consists of Type I Portland cement. And "hydraulic cement" refers also to cement that has the ability to set and harden when mixed with water. It is commonly used in construction and other applications where strong and durable structures are required. The hydraulic cement binder can be chosen from a variety of options, including: Portland Cement, the most common type of hydraulic cement used worldwide. It is made by grinding clinker (a mixture of calcium silicates) with gypsum. Type I Portland cement, also known as general-purpose cement, is commonly used for most construction applications; Blended Portland Cement, a type of cement that consists of a blend of Portland cement and supplementary cementitious materials such as fly ash, slag, or silica fume. The addition of these materials enhances certain properties of the cement, such as improved workability or reduced environmental impact; Expansive Cement, an expansive cement that undergoes volume expansion after hardening, compensating for shrinkage and minimizing cracking. It is useful in applications where restrained expansion is desired, such as in pre-stressed concrete; Rapid Setting and Hardening Cement, a type of cement designed to achieve high early strength development. It is commonly used in situations where quick setting and early strength gain are required, such as in cold weather concreting or emergency repairs; Calcium Aluminate Cement, a cement composed of calcium aluminates and known for its rapid setting and high-temperature resistance. It finds applications in specialized areas like refractory linings, industrial flooring, and sewage systems; Magnesium Phosphate Cement, a type of cement formed by mixing magnesium oxide and phosphate compounds. It has good chemical resistance and is often used in applications requiring rapid setting and high early strength, such as in certain repair works; Calcium Carbonate: a chemical compound with the formula $CaCO_3$. This is a common substance found in rocks, such as limestone, marble, and chalk. It has many industrial applications, including its use as a filler in the manufacturing of paper, plastic, and paints. It is also used in the construction industry as a building material and as an ingredient in cement; Dolomite-Based Material whereby dolomite is a mineral composed of calcium magnesium carbonate ($CaMg(CO_3)_2$) and whereby dolomite-based materials refer to materials that contain a significant amount of dolomite;

As used herein, the term "Curing" refers to the process of maintaining satisfactory temperature and moisture conditions in concrete for a period long enough for hydration to occur and concrete to develop the desired strength. The potential strength and durability of concrete will be fully developed only if concrete is properly cured or hardened. The cement-based materials, the concrete or the hydraulic cement can be cured or hardened or can be comprised of a material that is cured or hardened this way.

It's worth noting that while calcium carbonate and dolomite-based materials can be used as components in cement-based materials, they are not typically used alone as cured or hardened materials. Instead, they are often added as additives or fillers to modify certain properties of the final product.

Carbonation is the chemical reaction of carbon dioxide to give carbonates, bicarbonates, carbonic acid or any other chemical compound containing the anion written as $CO_3^{2-}$, called the carbonate anion.

A layered double hydroxide (LDH) is a material comprising hydroxides of a single or a multitude of monovalent ($M^I$), divalent ($M^{II}$), trivalent ($M^{III}$) and/or tetravalent ($M^{IV}$) metal cations intercalated by one or more kinds of anions ($A^{n-}$) and characterized by the general chemical formula $[M^1_z M^{II}_{1-x-y-z} M^{III}_x M^{IV}_y (OH)_2]^{x+2y-z} (A^{n-})_{n/(x+2y-z)} \cdot mH_2O$, where m is a real number. For instance, but not exhaustively, $M^I$ can be a single element of a combination of metals from $Li^+$, $K^+$. For instance, but not exhaustively, $M^{II}$ can be a single metal or a combination of metals from $Zn^{2+}$, $Mg^{2+}$, $Ni^{2+}$, $Ca^{2+}$, $Fe^{2+}$, $Co^{2+}$, $Cu^{2+}$, $Mn^{2+}$, $Pd^{2+}$, $Ti^{2+}$, $Cd^{2+}$. For instance, but not exhaustively, $M^{III}$ can be a single metal or a combination of metals from $Al^{3+}$, $Co^{3+}$, $Fe^{3+}$, $Mn^{3+}$, $Cr^{3+}$, $V^{3+}$, $Ce^{3+}$, $Dy^{3+}$, $Er^{3+}$, $Eu^{3+}$, $Gd^{3+}$, $Ho^{3+}$, $La^{3+}$, $Lu^{3+}$, $Nd^{3+}$, $Pr^{3+}$, $Pm^{3+}$, $Sm^{3+}$, $Sc^{3+}$, $Tb^{3+}$, $Tm^{3+}$, $Yb^{3+}$, $Y^{3+}$). For instance, but not restricted to, $M^{IV}$ can be $Ti^{4+}$.

A metal oxide is a material whose chemical formula is of the type $M_xO_y$, where x and y are real numbers, containing at least one kind of metal cation (M) and an oxide anion (O) and where x and y are real numbers. For instance, materials with chemical formula $MO$, $MO_2$, $M_2O_2$, $M_3O_2$. For instance, but not restricted to, M can be any or a combination from Zn, Mg, Ni, Ca, Fe, Co, Cu, Mn, Pd, Ti, Cd, Al, Co, Fe, Mn, Cr, V, Ce, Dy, Er, Eu, Gd, Ho, La, Lu, Nd, Pr, Pm, Sm, Sc, Tb, Tm, Yb, Y.

A mixed metal oxide is a metal oxide containing at least two different metal cations in its chemical formula.

A metal oxyhydroxide is a material whose chemical formula is of the type $M_xO_y(OH)_z$, containing at least one kind of metal cation (M) and an oxide anion (O) and where x, y and z are real numbers. For instance, but not restricted to, M can be any or a combination from Zn, Mg, Ni, Ca, Fe, Co, Cu, Mn, Pd, Ti, Cd, Al, Co, Fe, Mn, Cr, V, Ce, Dy, Er, Eu, Gd, Ho, La, Lu, Nd, Pr, Pm, Sm, Sc, Tb, Tm, Yb, Y.

A mixed metal oxyhydroxide is a metal oxyhydroxide containing at least two different metal cations in its chemical formula.

A lanthanide is an element from Ce, Dy, Er, Eu, Gd, Ho, La, Lu, Nd, Pr, Pm, Sm, Sc, Tb, Tm, Yb.

A lanthanide-doped LDH, a lanthanide-doped metal oxide, a lanthanide-doped mixed metal oxide and a lanthanide-doped mixed metal oxyhydroxide is, respectively, an LDH, a metal oxide, a mixed metal oxide and a mixed metal oxyhydroxide containing in its chemical formula at least one kind of lanthanide element.

An active sensor material is one or a combination of the following: a lanthanide-doped LDH, a lanthanide-doped metal oxide, a lanthanide-doped mixed metal oxide, a lanthanide-doped mixed metal oxyhydroxide.

Anions of interest for present invention are any of the following anions: carbonate ($CO_3^{2-}$), chloride ($Cl^-$), sulphate ($SO_4^{2-}$), nitrate ($NO_3^-$), nitrite ($NO_2^-$), $F^-$, $Br^-$, $I^-$, $I^{2-}$, $I^{3-}$, oxyanions of B, C, N, P, S, Cr, Mn, Ge, As, Se, Nb, Tc, Br, I, W, Re, Os, Ir, Pt, Hg and oxyanions of actinide elements (Th-Lr).

The photoluminescence properties of a material are, generally, the photoluminescence spectrum and the luminescence decay lifetime of the photoluminescence lines of a material.

The present invention concerns the use of an active sensor material, as defined above, to sense and quantify the presence of the anions of interest in alkaline materials. Further, the present invention concerns the use of the active sensor material combined with optical fibre detection of their luminescence properties for the sensing and quantification of anions of interest present in alkaline materials, here comprising any of 1) calcium carbonate or dolomite-based materials, 2) materials comprising calcium carbonate or dolomite, 3) cement-based materials or 4) concrete (for instance reinforced concrete). The detection and quantification of the anions of interest is hereby done by measuring the photoluminescence properties of an active sensor material in contact with said alkaline material to be monitored. During this contact, the active sensor material takes up (for instance, by chelation, intercalation and/or adsorption) anion of interest from said alkaline material. For instance, embedding an active sensor material in a concrete based structure (e.g., a structural pilar made of reinforced concrete) in conjunction with a set of optical fibres to excite active sensor material and read out its luminescence spectrum.

The object of present invention is to provide active sensor materials which are suitable to simultaneous fulfil the following: t:

> **A.** Feature long-term stability in hyper alkaline environment,
> **B.** Feature high affinity to the anions of interest,
> **C.** Be selective for the anion(s) to be sensed,
> **D.** Feature luminescence properties that change depending on the presence of the anion of interest in the medium to be assessed,
> **E.** Allow for absolute quantification of the anion of interest,
> **F.** Allow for quantification of the anion of interest without internal or external calibration, eliminating the need for measuring absolute luminescence intensities.

The present invention provides technical solutions making that lanthanide-doped layered double hydroxides and materials related to their calcination products (lanthanide doped mixed metal oxides and lanthanide doped mixed metal oxyhydroxides) fulfil all of the characteristics **A** to **F** mentioned above.

With respect to characteristic **A,** LDHs are materials formed at alkaline environments and thus are stable in concrete. *(A. A. Almeida, R. M. M. Santos, M. A. A. Rosa, S. H. Pulcinelli, V. M. John, and C. V. Santilli, ACS Appl. Nano Mater. 2022, 5, 6, 7896-7907)*

With respect to characteristics **B and C,** the anions in LDHs are exchangeable based on their affinity to the LDH host, this affinity increasing as follows: $CO_3^{2-}$ > $SO_4^{2-}$ > $F^-$ > $Cl^-$ > $Br^-$ > $NO_3^-$ > $I^-$, carbonate ($CO_3^{2-}$) standing in the beginning of this affinity series *(S. Miyata, Clays Clay Miner., 1983, 31, 305-311)*. LDHs, thus, have a high affinity and selectivity to carbonate, fulfilling **B** and **C.**

In general, the luminescence properties of lanthanide elements are mainly governed by the distance and symmetry of their first coordination shell, as well as by the polarizability of the ions directly coordinating to them. The introduction of lanthanides in the hydroxide layers of LDHs produces LDH whose luminescence properties depend on the anionic spacer's functional group. By functional group, it should be understood that a high degree of degeneracy is expected, and several anions lead to LDHs with same or similar luminescence properties. For instance, LDHs intercalated with carboxylates feature the same luminescence properties. (Silva, I. G. N., Morais, A. F., Lima, B. C., Garcia, F. A., & Mustafa, D. Applied Clay Science, 2020, 199, 105861*)*

Further, LDHs intercalated with β-diketones feature the same luminescence properties. (Morais, A. F., Machado, F. O., Teixeira, A. C., Silva, I. G. N., Breynaert, E., & Mustafa, D. Journal of Alloys and Compounds, 2019, 771, 578-583*)*

Further, it is often debated whether the luminescence properties of lanthanide doped materials are sensitive enough to be deemed useful to sense local compositional changes in host materials, since many chemical modifications lead to luminescence properties that are relatively close to each other. (Binnemans, K., & Görller-Walrand, C., Journal of Physics: Condensed Matter, 1998, 10(10), L167.). It is not expected that every lanthanide doped material can be used to probe carbonation in concrete as proposed in the present invention. Further, it is not expected that every anion can be probed, as many anions will lead to similar luminescence properties when they are intercalated in lanthanide doped LDHs. In general, LDHs and similar other materials, only partially fulfil characteristic **D.**

Further, characteristics **A-D** alone are not enough to solve the problems of the related art of assessing carbonation in concrete based structures. By fulfilling **A** to **D** alone, absolute quantification without external or internal calibration is impossible. The expected way to use luminescence as a sensing property is to detect absolute luminescence intensities. However, absolute luminescence intensities depend not only on the anion intercalated in the LDHs but also depend on practical parameters such as the concentration and possible dilution of LDHs on time inside concrete, how luminescence is measured, the efficiency of measuring and transmitting luminescence signals, the excitation intensity, the hydration state of the medium around the active sensor material, temperature, and pH, among others. (Melhuish, W. H. (1972). Journal of Research of the National Bureau of Standards. Section A, Physics and Chemistry, 76(6), 547). Further, inside a concrete structure, many of these parameters are likely to change during the service life of the structure. Measuring absolute luminescence intensities is thus not a solution for the related art. The changing conditions inside concrete structures render any method needing calibration useless for quantification of the degree of carbonation. A suitable solution is given by the present invention that allows to detect how much carbonate the LDH contains, without reference to an external or internal calibration, a feature that is key to the present invention.

Among the anions of interest as proposed for the present invention, nitrate ($NO_3^-$) and carbonate ($CO_3^{2-}$) are two inorganic anions that, when intercalated in lanthanide-doped LDHs, interact with the lanthanide element in a similar manner. These anions not only feature similar polarizability, (Lo, B. W. N. (1973). Journal of Physics and Chemistry of Solids, 34(3), 513-520) but they also interact with the lanthanide in LDHs through the same bond distance (our findings, not yet disclosed). It is thus unexpected that, as discovered and proposed by the inventors, intercalation with nitrate or carbonate produce lanthanide doped LDHs with strikingly different luminescence properties, as will be clear in the examples described below. This feature is key to the invention and makes lanthanide doped LDHs fulfil characteristic **D.**

When materials containing $Eu^{3+}$ are excited by ultraviolet radiation in the range from 390 - 400 nm, they emit a photoluminescence spectrum. In the spectral region from 580 to 710 nm, the photoluminescence spectrum of these materials shows the emission bands of $Eu^{3+}$, indicated by the term symbols of its 4f electronic states. In the said range, the spectrum features the $^5D_0 \rightarrow {}^7F_1$ and $^5D_0 \rightarrow {}^7F_1$ emissions. While the intensity $I(^5D_0 \rightarrow {}^7F_1)$ is independent on the host $Eu^{3+}$ is embedded in, the intensity $I(^5D_0 \rightarrow {}^7F_2)$ is sensitive to the geometry and chemical environment around the luminescent centre. By using the $I(^5D_0 \rightarrow {}^7F_2)/ I(^5D_0 \rightarrow {}^7F_1)$ intensity ratio instead of using the absolute luminescence intensity to sense and quantify carbonate and the other anions of interest, characteristics **E-F** can be fulfilled by proposed sensor material, which is an aspect of the present invention. The $I(^5D_0 \rightarrow {}^7F_2)/ I(^5D_0 \rightarrow {}^7F_1)$ intensity ratio is not dependent on practical parameters such as the concentration and possible dilution of LDHs on time inside concrete, how luminescence is measured, the efficiency of measuring and transmitting luminescence signals, the excitation intensity, the hydration state of the medium around the active sensor material, temperature, and pH. Thus, the use of this parameter and the findings that nitrate and carbonate intercalated in LDHs lead to strikingly different luminescence properties, as proposed by the present invention, are innovations that solve the problems described above and qualifies lanthanide doped LDHs to be used as calibrationless sensors to quantify carbonate and other anions of interest in concrete.

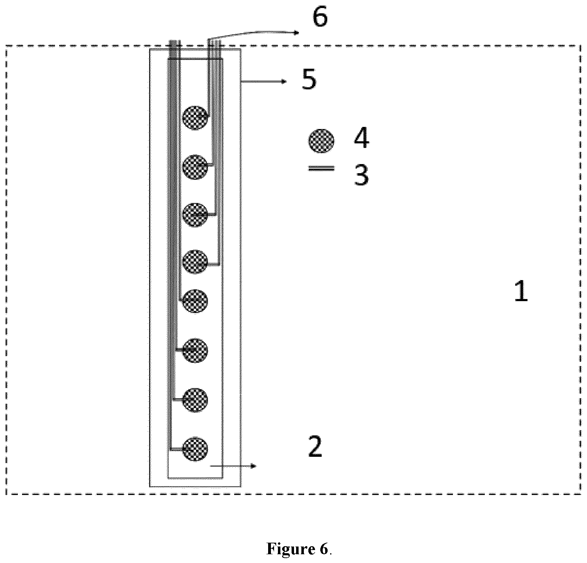

The present invention further concerns a sensor device (Figure 6) to be used for the sensing application described above for the active sensor material. The sensor device comprises, consists of or essentially consists of a mounting platform serving as fixation base for an active sensor material and for optical plugs where optical fibre bundles can be attached in order to allow monitoring the photoluminescence properties of the active sensor material. Further, each optical fibre bundle is a set of optical fibres with at least one optical fibre being connected, at one end, to a light emitting device serving as excitation source for the active sensor material. The remaining optical fibres in the bundle serve to collect the light emitted by the active sensor material. The optical fibre bundle is mounted in the mounting device in a way that the light emitted by the active sensor material after excitation reaches the end of the optical fibre that is attached to the mounting device. From the outside of the built structure, a light emitting device serving as excitation source and a fluorimeter (e.g. a portable fluorimeter) can be connected to the respective excitation and measurement optical fibres whenever the carbonation of the structure is intended to be assessed.

The examples shown below are intended to demonstrate some of the many ways to carry out the invention and provide some nomenclature used herein. These examples, however, do not limit the scope of the invention, which can be implemented in other specific ways, without deviating from the essential attributes to be described by the claims.

## EXAMPLES

As an non-exhaustive example of realization of such an invention, the carbonation of reinforced concrete (one possible anion of interest), here meant by the presence of carbonate ($CO_3^{2-}$) in concrete, can be monitored and quantified by the changes in the photoluminescence properties of layered double hydroxides with original formula [$Zn_2Al_{0.95}Eu_{0.05}$ $(OH)_6]^+(NO_3^-).mH_2O$ that undergoes partial or total replacement of $NO_3^-$ by $CO_3^{2-}$ when immersed in concrete containing $CO_3^{2-}$. The examples described below are intended to clarify the realization of such an application and a carbonate sensor device employing said LDH as active sensor device. Further, the following examples are intended to make the invention clear to those skilled in the art from consideration of the specification and practice of the invention disclosed herein.

**Example 1:** Synthesis of an Eu-doped LDH

An Eu$^{3+}$-doped LDH containing Zn$^{2+}$ and Al$^{3+}$ and nitrate (NO$_3$$^-$) at the proportions specified by the chemical formula [Zn$_2$Al$_{0.95}$Eu$_{0.05}$(OH)](NO$_3$$^-$).mH$_2$O, where 0 < m < 1, herein LDH-N, is a lanthanide-doped LDH that can be prepared as follows. A ten-millilitres aqueous solution (solution A) containing Zn(NO$_3$)$_2$, Al(NO$_3$)$_3$ and Eu(NO$_3$)$_3$ at concentrations of 0.66, 0.314 and 0.016 mol.L$^{-1}$, respectively, is added dropwise (10 mL.h$^{-1}$) into 200 mL of Milli-Q water (solution B). During the dosing, the pH of solution B is kept in the range 7.8-8.2 by the addition of a 1 mol.L$^{-1}$ NaOH solution by an automatic titrator (e.g., Metrohm 785 DMP). After the end of the dosing, the result is aged statically for two days at 60 °C and subsequently centrifuged, rinsed with distilled water and dried in a drying oven at 60 °C for 3 days. The final solid is ground with mortar and pestle to produce a fine powder. For the synthesis of an LDH with chemical formula [Zn$_2$Al$_{0.95}$Eu$_{0.05}$(OH)](CO$_3$$^{2-}$)$_{0.5}$.mH$_2$O, herein LDH-C, the same procedure was repeated, but solution B comprised an aqueous solution of Na$_2$CO$_3$ 0.5 mol.L$^{-1}$.

**Example 2:** Anion exchange and production of Eu-doped LDHs containing a mixture of nitrate and carbonate

To produce an Eu-doped LDH containing a mixture of nitrate (NO$_3$$^-$) and carbonate (CO$_3$$^{2-}$), 300 mg of pristine LDH-N can be suspended in 5 mL of a Na$_2$CO$_3$ aqueous solution whose concentration will determine the fraction anions X = CO$_3$$^{2-}$/(NO$_3$$^-$ + CO$_3$$^{2-}$) of carbonate anions intercalated in the LDH in the end of the procedure. The procedure can follow by stirring the suspension for 24 h and, after that, centrifuging the result and washing the rinsing the result with 100 mL of deionized water. The product is dried at 60 °C for 3 days. For instance, by using concentrations of Na$_2$CO$_3$ of 20, 40, 60, 80, 100 and 200 mmol.L$^{-1}$, samples herein referred to, respectively, LDH-20, LDH-24, LDH-60, LDH-80, LDH-100 and LDH-200 are obtained with a fraction X of, respectively, 0.5, 0.65, 0.87, 0.95, 1.0 and 1.0.

**Example 3:** Methods used herein for characterization of materials

Powder X-ray diffraction (PXRD) was performed in a Bruker D8 (Massachusetts, EUA) diffractometer (CuK$\alpha$, 1.5418 Å). For Carbon, Hydrogen and Nitrogen (CHN) elemental analysis, a Perkin Elmer (Massachusetts, EUA) 2400 series ii elemental analyser was used. The *ex situ* photoluminescence measurements were performed in a FS5 Spectrofluorometer (Edinburgh Instruments) spectrofluorometer with a 450 W Xenon lamp equipped with singular monochromators both at the excitation and the emission optics. All spectra shown were corrected for the intensity profile of the Xenon lamp and detection response.

**Example 4.** Method used herein for *in situ* photoluminescence measurements:

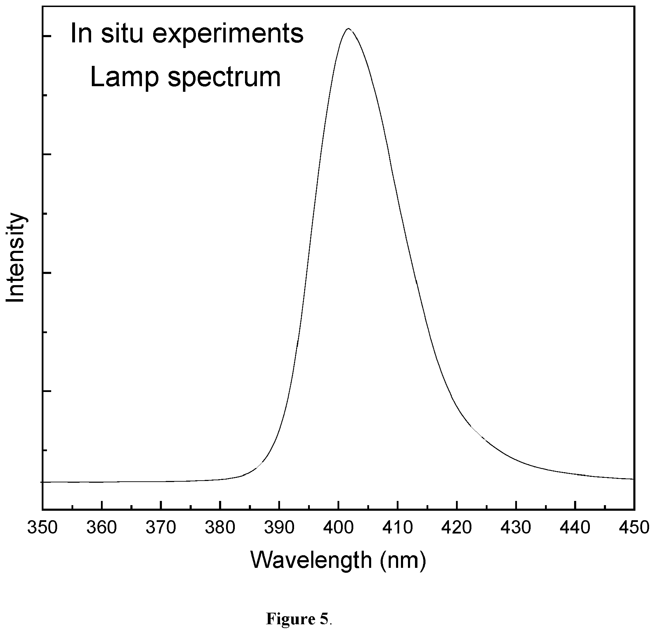

For the in situ measurements of the luminescence properties of LDHs during anion exchange reactions, 300 mg of pristine LDH-N were suspended in 5 mL of a Na$_2$CO$_3$ aqueous solutions at concentrations of 20, 100 and 200 mmol.L$^{-1}$. The LDHs used in the different suspensions originated from the same batch. To follow the luminescence properties of LDHs *in situ* during the anion exchange reaction, a setup comprising an Ocean Optics USB2000 portable fluorimeter equipped with an optical fibre to monitor the emission of the sample was assembled. A commercial 10 W UV LED lamp (Figure 5) was used as excitation source and was place on top of the suspension.

**Example 5.** Structural and photoluminescence properties of the Eu$^{3+}$-doped LDHs:

The structural and luminescent properties of Eu-containing Zn$^{2+}$/Al$^{3+}$ LDHs were investigated. The changes in the photoluminescence properties (PL) of these LDHs have been followed in the equilibrium state and in situ during anion exchange reactions where pristine LDHs initially intercalated with nitrate (LDH-N) were suspended in sodium carbonate solutions at different concentrations. These anion exchange reactions led to LDHs where nitrate was completely or partially exchanged to carbonate, which could be qualitatively and quantitatively assessed by, respectively, powder X-ray diffraction (PXRD) and elemental analysis. LDHs with remarkably different luminescence properties have been obtained after the anion exchange reactions. Europium coordination was analysed by Extended X-ray Absorption Fine Structure (EXAFS) spectroscopy.

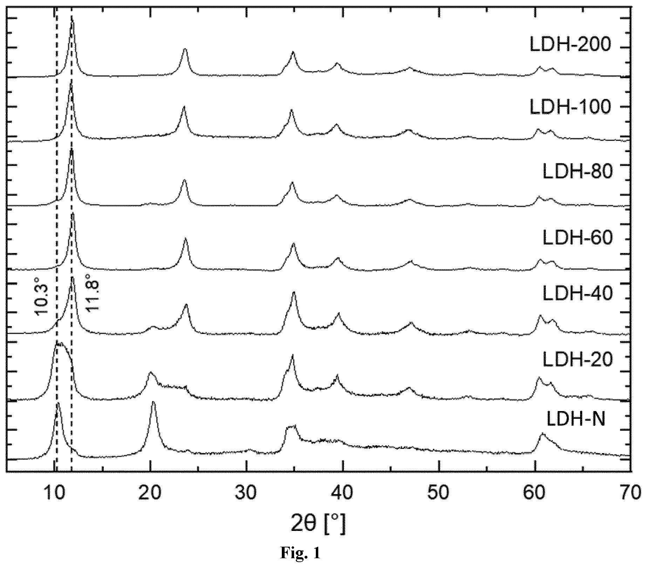

In brief, the inclusion of Eu$^{3+}$ in the hydroxide layers of LDHs can be done through synthetic procedures involving controlled hydrolysis (see Example 1), yielding pristine nitrate intercalated LDHs with a characteristic interlayer distance of 8.58 Å as revealed by the (003) Bragg reflection at 10.3° 2θ in their PXRD pattern (Figure 1).

Carbonate sequestration with release of nitrate when pristine LDH-N is suspended in a Na$_2$CO$_3$ solution is evident both from elemental analysis (Table 2) and PXRD (Figure 1). Carbonate intercalated LDHs feature a shorter interlayer distance as compared to nitrate intercalated LDHs. This makes a basal reflection to appear at 11.8° 2θ for the carbonate intercalated materials.

### Example 6. Sensor device

A sensor device could consist of an assembly of multiple sensor elements positioned on a mounting platform, not only serving as support for the active sensor material (the LDH), but also as positioning device for a set of optical fibre bundles connecting the sensor to the outside world. An example of a device whose concept is described herein can be realized by a mounting device serving as fixation base for the active sensor material and for optical plugs where fibre bundles can be attached. Examples of active sensor materials are lanthanide doped layered double hydroxides (LDH), lanthanide doped metal oxides and lanthanide doped double metal oxyhydroxides. Examples of presentations of the active sensor material encompasses but are not restricted to pilules, sachets, loosely packed powders or pressed pellets. Each fibre bundle is a set of optical fibres with at least one fibre being connected, at one end, to a light emitting device serving as excitation source for the lanthanide hosted in the active sensor material. The remaining fibres in the fibre bundle serve to collect the light emitted by the active sensor material. The fibre bundle should be mounted in the mounting device in a way that the light emitted by the active sensor material reaches the end of the fibre that is attached to the mounting device. One example of a suitable alignment is the fibre bundle pointing directly to the active sensor material. In case of an active sensor material that is doped with a $Eu^{3+}$, a LED device emitting in the band from 380 and 410 nm is a suitable excitation source to be connected to the (dangling) excitation end of the fibre bundle. For the same carbonate sensor working with a $Eu^{3+}$-doped active sensor material, a set of optical fibres working in the spectral range from 550 to 750 nm is an example of suitable optical fibre serving to collect the light emitted by the active sensor material after excitation at the proper excitation band.

In the workable example described above, the whole sensor device comprising the mounting device, the active sensor material, the porous encasing, the optical fibre bundles, and the connector plugs is immersed in the built structure. For the specific application of assessing the evolution of the carbonation degree in reinforced concrete, the sensor device can be immersed in the concrete during concrete pouring, letting the dangling end of the fibre bundles outside the concrete structure in a way that this end can be connected to a fluorimeter (e.g. a portable fluorimeter) whenever the carbonation of the structure needs to be assessed.

Measurement practice: the carbonation degree in the position of each spot containing the active sensor material can be measured by connecting a fluorimeter and an excitation source (suitable to the active sensor material in use) to the end of the fibre bundle that is opposed to the spot to be measured. Upon collection of the spectrum emitted by the active sensor material, the carbonation degree can be calculated in a calibrationless manner.

**Example 6:** Example data analysis:

Table 2 shows the elemental composition of LDH-N before and after anion exchange. The Carbon and Nitrogen content in the samples and the substitution fraction (X) as read in the LDH general formula $[Zn_2Al_{0.95}Eu_{0.05}(OH)_6][(NO_3^-)_{1-X}(CO_3^{2-})_{X/2}]\cdot mH_2O)$ is presented. Nitrate was exchanged to carbonate by suspending LDH-N in solutions of different initial carbonate concentrations (samples dubbed LDH-A, A = 20, 40, 60, 80, 100 and 200 standing for the initial carbonate concentration, in $mmol.L^{-1}$, in the anion exchange solution). In line with the PXRD results, the Carbon content in the samples increases with the increase in the carbonate concentration in the exchange solution, confirming that LDHs are effective materials for carbonate uptake. At the same time, the nitrogen content decreases, showing that carbonate is fixed in the samples at the expense of the nitrate anions. From the point of view of elemental analysis, carbonate saturation in the LDHs is reached for initial carbonate concentrations between 80 (X = 0.95) and 100 $mmol.L^{-1}$ (X = 1.0). Interestingly, as seen from PXRD, carbonate already dominates the interlayer space of the LDHs for concentrations above 60 $mmol.L^{-1}$. Above this concentration, the structural properties of the LDHs hinders any tentative to discriminate the presence or absence of nitrate by using PXRD.

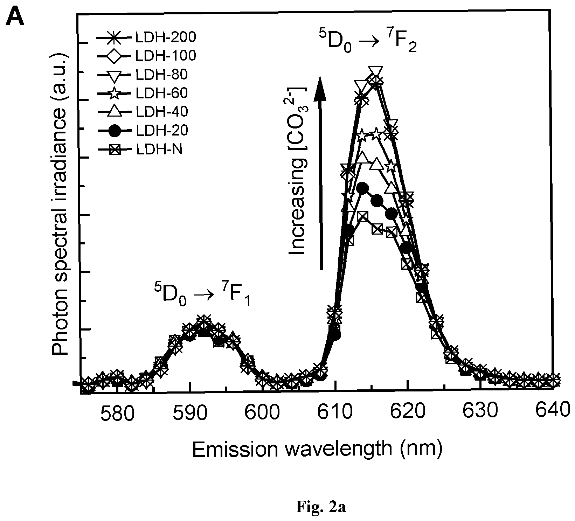

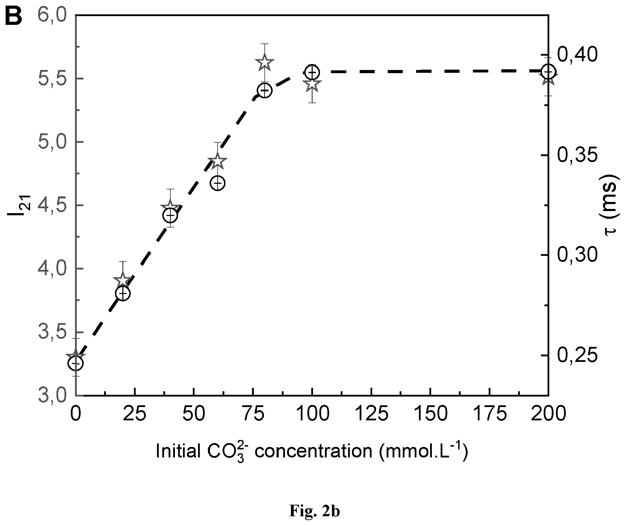

The PL spectra obtained for LDH-A (A = A = 20, 40, 60, 80, 100 and 200) are shown on Figure 2A. In the spectra shown on Figure 2A, all spectral profiles have been normalized by the integral intensity $I(^5D_0\rightarrow^7F_1)$. With this internal normalization, the $(Eu^{3+})^5D_0\rightarrow^7F_2$ emission has been observed to increase (Figure 2B-C) with the $CO_3^{2-}$ concentration in solution. While the PXRD patterns of LDH-60 and LDH-80 are very similar, the $I_{21} = I(^5D_0\rightarrow^7F_2)/I(^5D_0\rightarrow^7F_1)$ intensity ratio of these two samples are strikingly different, as seen from Figure 2B, thus showcasing the sensitivity of the luminescence properties of Eu-containing LDH to determine differences in the carbonate content between two different LDH hosts. Also the decay lifetime (Figure 2B-C and Table 1) of the $(Eu^{3+})^5D_0\rightarrow^7F_2$ transition has been observed to increase with the uptake of carbonate from the anion exchange solution. With carbonate concentrations exceeding 100 $mmol.L^{-1}$ these changes cease, indicating the saturation of the anion sites in the LDHs, which is consistent with the stoichiometry derived from elemental analysis (Table 2).

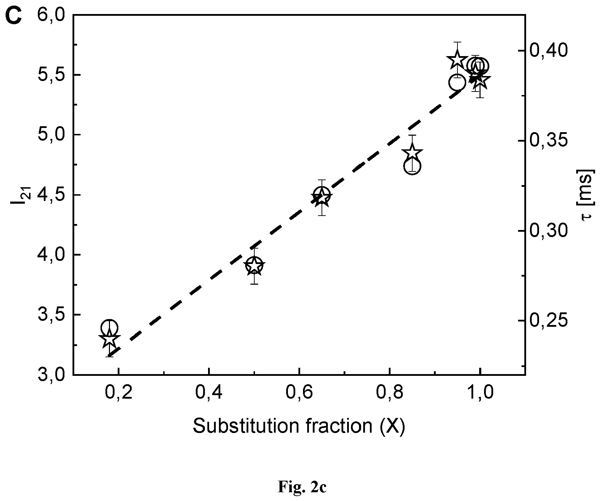

Both the luminescence decay lifetime and the $I(^5D_0\rightarrow^7F_2)/I(^5D_0\rightarrow^7F_1)$ intensity ratio in the Eu-containing LDHs strongly correlate with the carbonate load (X) in the samples, as shown in Figure 2C. The $I(^5D_0\rightarrow^7F_2)/I(^5D_0\rightarrow^7F_1)$ intensity ratio is shown to feature a linear relationship with the carbon concentration in the interlayer gallery.

In the example of an active sensor material comprised by $Eu^{3+}$-doped ZnAl LDHs originally intercalated by nitrate anions (LDH-N), the carbonation degree can then be quantified by the amount of carbonate taken up by the active sensor material as given by the following equation:

$$wt\%\,C = 0.75 \times (I_{21} - 2.8),$$

where $I_{21} = I(^5D_0 \rightarrow {}^7F_2)/\,I(^5D_0 \rightarrow {}^7F_1)$ is the ratio between the integral intensities of the $^5D_0 \rightarrow {}^7F_2$ and $^5D_0 \rightarrow {}^7F_1$ emission bands of $Eu^{3+}$. wt%C ranges from 0 to approximately 2%, this maximum figure meaning that the sensor is saturated. This correlation can then be used for *in situ* measurements of the carbonate loading in the LDHs.

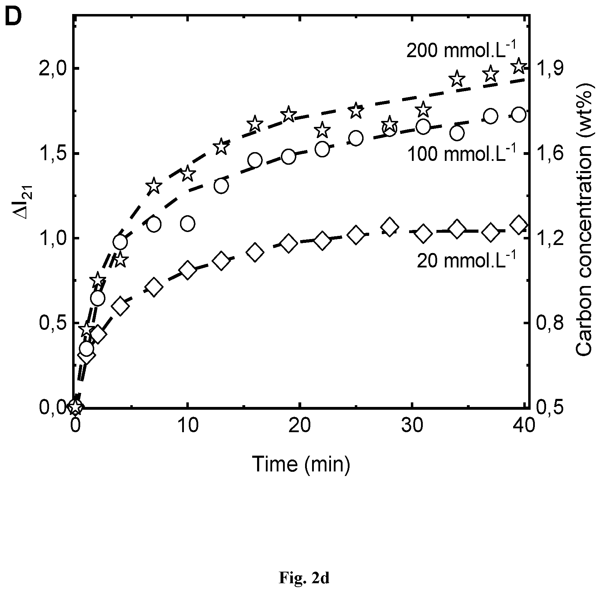

To follow the luminescence properties of LDHs *in situ* during a carbonate uptake experiment, a setup comprising a portable fluorimeter equipped with an optical fibre to monitor the emission of the sample was assembled. A commercial 10 W UV LED lamp was used as excitation source. Carbonate solutions with three different concentrations were added to three LDH suspensions, with the LDHs used in the different suspensions originated from the same batch. For these *in situ* experiments, the initial carbonate concentrations were chosen to be well below (20 mmol.L$^{-1}$), similar to (100 mmol.L$^{-1}$) and well beyond (200 mmol.L$^{-1}$) the concentration seen by elemental analysis to cause saturation of the anionic sites in the LDHs. As evident from **Figure 2D,** the carbonate uptake by the LDHs is followed by an increase in time of the $I(^5D_0 \rightarrow {}^7F_2)/I(^5D_0 \rightarrow {}^7F_1)$ intensity ratio. Eq. 1 was used to calculate the carbonate concentration in the solid phase based on the measured luminescence properties. The carbonate load in the solid phase linearly increases in time in the initial steps of the exchange reaction. Then, as equilibrium is reached, a plateau starts to form, with the stabilization of the carbonate loading in the solid phase. Similar carbonate uptake profiles are observed for the LDHs suspended in the solutions with initial carbonate concentration of 100 and 200 mmol.L$^{-1}$, again a consequence of the saturation of the LDHs.

In summary, the here reported experiments show that the introduction of $Eu^{3+}$ in the hydroxide layers of layered double hydroxides enables the optical assessment of the degree of carbonate uptake by the LDH matrix. Since the intensity of the magnetic dipole transition $(Eu^{3+})^5D_0 \rightarrow {}^7F_1$ is largely independent on the host, it can serve as an internal standard during the analysis of PL spectra, turning $Eu^{3+}$-doped LDHs into calibrationless carbonate sensors with potential application for *in situ,* non-destructive assessment of the progress of the carbonation front in concrete. This would enable continuous, remote quality control of critical infrastructure built from reinforced concrete.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

**Drawing Description**

**BRIEF DESCRIPTION OF THE DRAWINGS**

The present invention will become more fully understood from the detailed description given herein below and the accompanying drawings which are given by way of illustration only, and thus are not limitative of the present invention, and wherein:

FIG. 1 is a graphic showing the PXRD patterns of the as-prepared LDH-N and the anion exchanged LDH samples. Samples are named after the initial carbonate concentration in mmol.L$^{-1}$ in the solution used in the anion exchange process (LDH-A, A = 20, 40, 60, 80, 100 and 200). The nitrate- and carbonate-intercalated phases can be identified by the characteristic basal reflections at 10.3 and 11.8° 2θ, respectively.

FIG. 2 is a graphic showing the emission spectra (A), $I_{21} = I(^5D_0 \rightarrow {}^7F_2)/\,I(^5D_0 \rightarrow {}^7F_1)$ intensity ratio (B, ☆, left scale) and luminescence decay lifetime (B, ○, right scale) of the anion exchanged LDHs after equilibration at different concentrations of $CO_3^{2-}$. The spectra are normalized by the integral intensity of the $(Eu^{3+})^5D_0 \rightarrow {}^7F_1$ emission band. (C) $I_{21}$ intensity ratio (*, left scale) and luminescence decay lifetime (○, right scale) as a function of the carbonate substitution fraction (X) in the LDH samples after equilibration. (D) Changes in the $I_{21}$ intensity ratio over time in the in-situ experiments with different initial carbonate concentrations. The right scale shows the carbon concentration calculated from Eq. 1.

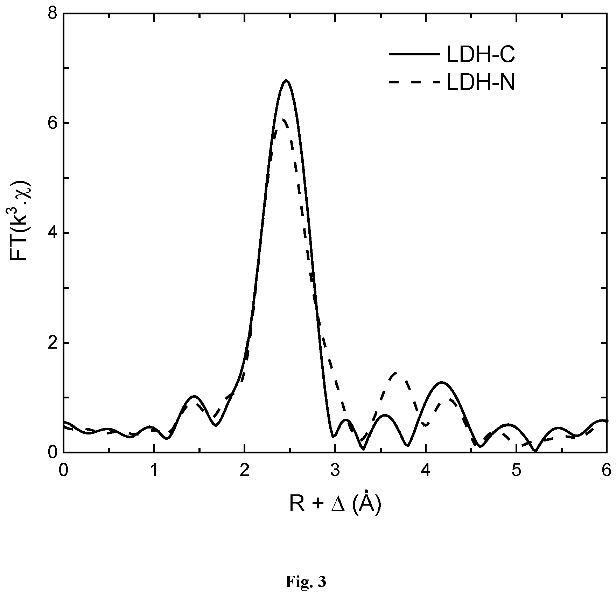

FIG. 3 is a graphic showing phase-corrected Fourier transform of the Eu LIII-edge EXAFS data for LDH-N and a carbonate-intercalated $Zn^{2+}/Al^{3+}/Eu^{3+}$ LDH, named LDH-C.

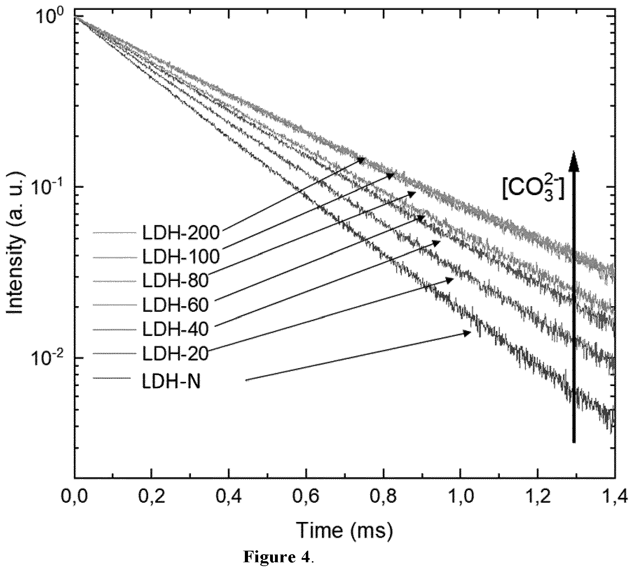

FIG. 4 is a graphic showing luminescence decay curves monitored on the $(Eu^{3+})^5D_0 \rightarrow {}^7F_2$ emission band after direct excitation of the $Eu^{3+}$ activators (394 nm).

FIG. 5 is a graphic showing a spectrum of the UV lamp used as excitation source for the *in situ* luminescence measurements.

FIG. 6 shows an example of a sensor design for built structure 1, e.g., the concrete part of a build structure 1, whereon the sensor material 4 of present invention is mounted in accordance with the invention, with the mounting device 2, the sensing material 4, optical fibre bundles 3, connecting the sensing material 4 with an excitation source and fluorimeter 6. Optionally a porous encasing 5 surrounds the sensor material 4 and at least part of the optical fibre bundles 3. Optic fibres 3 are functionally connect with sensing materials 4.

**TABLES**

**Table 1.** Luminescence decay lifetime.

| Sample | Lifetime (ms) | Sample | Lifetime (ms) |
|---|---|---|---|
| ZnAlEu-N | 0.246(3) | LDH-80 | 0.382(3) |
| LDH-20 | 0.281(3) | LDH-100 | 0.391(3) |
| LDH-40 | 0.320(3) | LDH-200 | 0.392(3) |
| LDH-60 | 0.336(3) | | |

**Table 2.** Experimental chemical composition of pristine $LDH-NO_3$ and LDH-A (A = 20, 40, 60, 80, 100 and 200).

| Sample | %C | %N | Empirical formula |
|---|---|---|---|
| LDH-N | 0.34 | 3.42 | $[Zn_2Al_{0.95}Eu_{0.05}(OH)_6][(NO_3^-)_{0.82}(CO_3^{2-})_{0.09}] \cdot nH_2O$ |
| LDH-20 | 0.95 | 2.20 | $[Zn_2Al_{0.95}Eu_{0.05}(OH)_6][(NO_3^-)_{0.50}(CO_3^{2-})_{0.25}] \cdot nH_2O$ |
| LDH-40 | 1.22 | 1.50 | $[Zn2Al_{0.95}Eu_{0.05}(OH)_6][(NO_3^-)_{0.35}(CO_3^{2-})_{0.325}] \cdot nH_2O$ |
| LDH-60 | 1.68 | 0.25 | $[Zn2Al_{0.95}Eu_{0.05}(OH)_6][(NO_3^-)_{0.13}(CO_3^{2-})_{0.435}] \cdot nH_2O$ |
| LDH-80 | 1.86 | 0.18 | $[Zn_2Al_{0.95}Eu_{0.05}(OH)_6][(NO_3^-)_{0.05}(CO_3^{2-})_{0.475}] \cdot nH_2O$ |
| LDH-100 | 2.20 | 0.01 | $[Zn_2Al_{0.95}Eu_{0.05}(OH)_6][(CO_3^{2-})_{0.5}] \cdot nH_2O$ |
| LDH-200 | 2.02 | 0.06 | $[Zn_2Al_{0.95}Eu_{0.05}(OH)_6][(NO_3^-)_{0.01}(CO_3^{2-})_{0.495}] \cdot nH_2O$ |

**Claims**

1. A method to monitor, to analyse or to quantify the presence of an anion of interest into a built structure or part thereof, whereby the built structure or part thereof to be dosed comprises a cured or hardened material selected of the group consisting of calcium carbonate and dolomite based material, materials comprising calcium carbonate or dolomite, cement based materials and concrete, whereby the method comprising said contacting the built structure or part thereof with an active sensor material comprising a lanthanide doped material of the group consisting of layered double hydroxides (LDH), metal oxides, mixed metal oxides, mixed metal oxyhydroxides and sensing the fluorescent signal thereof to sense the anion of interest.

2. The method according to claim 1, whereby the active sensor material comprises $Eu^{3+}$-doped layered double hydroxide (LDH).

3. The method according to any one of the claims 1 and 2, whereby the fluorescent signal sensor is an optical sensor.

4. The method according to any one of the claims 1 to 3, whereby the sensor further comprising a fluorimeter coupled to receive the fluorescent signal and a controller coupled to fluorimeter that transmits the signal to a processor to process the luminescence decay lifetime and/or the luminescence intensity in the sensor into a quantification of load of the anion of interest.

5. The method according to any one of the claims 1 to 3, whereby the sensor further comprising a fluorimeter coupled to receive the fluorescent signal and a controller coupled to fluorimeter that transmits the signal to a processor to process the luminescence decay lifetime and/or the $I(^5D_0{\rightarrow}^7F_2)/ I(^5D_0{\rightarrow}^7F_1)$ luminescence intensity ratio in the active sensor into a quantification of carbonate load whereby the anion intrusion into a built structure is quantified without reference to an external calibration

6. The method according to any one of the claims 1 to 5, whereby the cured or hardened material is cured or hardened reinforced concrete.

7. The method according to any one of the claims 1 to 5, whereby the cured or hardened material is cured or hardened reinforced concrete comprising Porland cement.

8. The method according to any one of the claims 1 to 5, whereby the anion of interest is any one of the group consisting of carbonate ($CO_3^{2-}$), chloride ($Cl^-$), sulphate ($SO_4^{2-}$), nitrate ($NO_3^-$) and nitrite ($NO_2^-$).

9. The method according to any one of the claims 1 to 5, whereby the anion of interest is any one of the group consisting of carbonate $F^-$, $Br^-$, $I^-$, $I^{2-}$, $I^{3-}$, oxyanions of B, C, N, P, S, Cr, Mn, Ge, As, Se, Nb, Tc, Br, I, W, Re, Os, Ir, Pt, Hg and oxyanions of actinide elements (Th-Lr).

10. The method according to any one of the claims 1 to 5, whereby the intruding anions are of the group of Cl- and $CO_3^{2-}$ or a combination thereof.

11. The method according to any one of the claims 1 to 5, whereby the anion of interest is carbonate ($CO_3^{2-}$) and whereby a processor quantifies the carbonation degree by the amount of carbonate taken up by an $Eu^{3+}$-doped active sensor material by the equation: $wt\% \, C = 0.75 \times (I_{21} - 2.8)$, where $I_{21} = I(^5D_0 \rightarrow {}^7F_2)/ I(^5D_0 \rightarrow {}^7F_1)$ is the ratio between the integral intensities of the $^5D_0 \rightarrow {}^7F_2$ and $^5D_0 \rightarrow {}^7F_1$ emission bands of $Eu^{3+}$ in an Eu-containing active sensor material. By carbonate uptake, wt%C increases ranging from 0 to approximately 2%, this maximum figure meaning that the sensor is saturated.

12. The method according to any one of the claims 1 to 11, to monitor the changing chemical environment of the built structure during monitoring session of years to analyse the integrity of the built structure.

13. The method according to any one of the claims 1 to 11, to monitor the changing chemical environment of the built structure during monitoring session of decades to analyse the integrity of the built structure.

14. The method according to any one of the claims 1 to 11, to monitor the changing chemical environment of the built structure.

15. The method according to any one of the claims 1 to 11, to monitor the built structure by measuring absolute luminescence intensities.

16. The method according to any one of the claims 1 to 11, to monitor the built structure by measuring absolute luminescence intensities and calculating luminescence lifetimes from consequent measurements.

17. The method according to any one of the claims 1 to 11, whereby the active sensor contact said cured or hardened built structure to take up carbonate that is present in the cured or hardened material and the resulting photoluminescence of the active sensor material is measured.

18. The method according to any one of the claims 1 to 17, whereby the active sensor is embedded in the cured or hardened material of the built structure.

19. The method according to any one of the claims 1 to 18, whereby the active sensor is functionally connected with optical fibres which are connected or connectable with a fluorimeter.

Fig. 1

Fig. 2a

**Fig. 2b**

**Fig. 2c**

**Fig. 2d**

Fig. 3

Figure 4.

**Figure 5**.

**Figure 6**.

Europäisches
Patentamt

European
Patent Office

Office européen
des brevets

# EUROPEAN SEARCH REPORT

Application Number

EP 23 17 4755

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 2015 184215 A (CENTRAL RES INST ELECT) 22 October 2015 (2015-10-22) * paragraph [0001] * * paragraphs [0005], [0006] * * paragraphs [0016] - [0020] * * figure 1 * | 1-15 | INV. G01N31/22 G01N21/64 G01N21/78 G01N33/38 |
| A | JP 2015 121470 A (UNIV IWATE) 2 July 2015 (2015-07-02) * paragraphs [0003] - [0005] * * paragraphs [0010] - [0012] * * paragraphs [0018] - [0021] * * paragraph [0021]; figures 2-5 * * paragraph [0035] * | 1-15 | |
| A | US 2002/057095 A1 (ZOUGHI REZA [US] ET AL) 16 May 2002 (2002-05-16) * paragraphs [0003] - [0005] * * paragraphs [0009] - [0011] * * paragraph [0028] * * paragraphs [0055] - [0056]; figures 26-27 * | 1-15 | |
| A | US 2016/258881 A1 (BOWN MARK [AU] ET AL) 8 September 2016 (2016-09-08) * paragraphs [0002], [0003] * * paragraph [0008] * * paragraph [0023] * | 1-15 | TECHNICAL FIELDS SEARCHED (IPC) G01N |
| A | CN 101 726 525 A (HARBIN INST OF TECHNOLOGY) 9 June 2010 (2010-06-09) * the whole document * | 1-15 | |

The present search report has been drawn up for all claims

| Place of search | Date of completion of the search | Examiner |
|---|---|---|
| Munich | 26 February 2024 | Michalitsch, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 17 4755**

This annex lists the patent family members relating to the patent documents cited in the above-mentioned European search report.
The members are as contained in the European Patent Office EDP file on
The European Patent Office is in no way liable for these particulars which are merely given for the purpose of information.

**26-02-2024**

| Patent document cited in search report | | Publication date | Patent family member(s) | | Publication date |
|---|---|---|---|---|---|
| JP 2015184215 | A | 22-10-2015 | JP | 6249850 B2 | 20-12-2017 |
| | | | JP | 2015184215 A | 22-10-2015 |
| JP 2015121470 | A | 02-07-2015 | JP | 6338238 B2 | 06-06-2018 |
| | | | JP | 2015121470 A | 02-07-2015 |
| US 2002057095 | A1 | 16-05-2002 | NONE | | |
| US 2016258881 | A1 | 08-09-2016 | AU | 2006246300 A1 | 16-11-2006 |
| | | | BR | PI0609385 A2 | 18-10-2011 |
| | | | CA | 2607513 A1 | 16-11-2006 |
| | | | CN | 101208619 A | 25-06-2008 |
| | | | CN | 102818793 A | 12-12-2012 |
| | | | CN | 104463295 A | 25-03-2015 |
| | | | EP | 1880236 A1 | 23-01-2008 |
| | | | JP | 2008541064 A | 20-11-2008 |
| | | | JP | 2012123004 A | 28-06-2012 |
| | | | JP | 2014206537 A | 30-10-2014 |
| | | | JP | 2016188865 A | 04-11-2016 |
| | | | NZ | 563127 A | 24-12-2010 |
| | | | US | 2009084981 A1 | 02-04-2009 |
| | | | US | 2016258881 A1 | 08-09-2016 |
| | | | WO | 2006119561 A1 | 16-11-2006 |
| | | | ZA | 200709693 B | 29-10-2008 |
| CN 101726525 | A | 09-06-2010 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HERBERT PÖLLMANN** ; **RUBEN SNELLINGS** ; **LUCA VALENTINI**. Cement and Concrete-Past, Present, and Future. *Elements*, 2022, vol. 18 (5), 295-299
- **P. C. ASSOCIATION**. Types and causes of concrete deterioration. *Portl. Cem. Assoc. - Concr. Inf.*, 2002
- **DE MAEIJER, P. K.** ; **CRAEYE, B.** ; **SNELLINGS, R.** ; **KAZEMI-KAMYAB, H.** ; **LOOTS, M.** ; **JANSSENS, K.** ; **NUYTS, G.** Effect of ultra-fine fly ash on concrete performance and durability. *Construction and Building Materials*, 2020, vol. 263, 120493
- **A. A. ALMEIDA** ; **R. M. M. SANTOS** ; **M. A. A. ROSA** ; **S. H. PULCINELLI** ; **V. M. JOHN** ; **C. V. SANTILLI**. *ACS Appl. Nano Mater.*, 2022, vol. 5 (6), 7896-7907
- **S. MIYATA**. *Clays Clay Miner.*, 1983, vol. 31, 305-311
- **SILVA, I. G. N.** ; **MORAIS, A. F.** ; **LIMA, B. C.** ; **GARCIA, F. A.** ; **MUSTAFA, D.** *Applied Clay Science*, 2020, vol. 199, 105861
- **MORAIS, A. F.** ; **MACHADO, F. O.** ; **TEIXEIRA, A. C.** ; **SILVA, I. G. N.** ; **BREYNAERT, E.** ; **MUSTAFA, D.** *Journal of Alloys and Compounds*, 2019, vol. 771, 578-583
- **BINNEMANS, K.** ; **GÖRLLER-WALRAND, C.** *Journal of Physics: Condensed Matter*, 1998, vol. 10 (10), L167
- **MELHUISH, W. H.** *Journal of Research of the National Bureau of Standards. Section A, Physics and Chemistry*, 1972, vol. 76 (6), 547
- **LO, B. W. N.** *Journal of Physics and Chemistry of Solids*, 1973, vol. 34 (3), 513-520